# EUROPEAN PATENT APPLICATION

(11) **EP 4 108 094 A1**
(43) Date of publication of application: **28.12.2022**
(21) Application number: 21181254.0
(22) Date of filing: 23.06.2021
(51) Int. Cl.: A23F 5/24, A23F 5/26, A23L 33/105, A61K 8/9789, A61K 36/74

(54) **COFFEE MELANOIDIN NANOPARTICLES AND NANOFIBERS**

(71) Applicant: Kaffee Bueno ApS, 2860 Søborg (DK)
(72) Inventor: Ascensão Aroso, Ivo Manuel, 4700-103 Braga (PT); de Sá Bessa, Ricardo, 4450-023 Matosinhos (PT); Gonçalves dos Reis, Rui Luis, 4250-242 Porto (PT); Akcay, Adnan, 2860 Søborg (DK); Medina, Juan, 2860 Søborg (DK)
(74) Representative: De Vries & Metman

(57) **Abstract**

The invention relates to a process for preparing a melanoidin product, the process comprising:
a) an extraction step in which coffee grounds are treated with an extraction agent with a pH value greater than 7 to extract a melanoidin-containing solute in a fluid phase of the extraction agent;
b) a first separation step in which the fluid phase is separated from the coffee grounds;
c) a precipitation step in which the fluid phase is contacted (i) with an acid to obtain an acidic mixture with a pH value lower than 4 or (ii) with an organic phase separation agent and with an acid to obtain a mixture with a pH value in the range of from 4 to 8, thereby forming precipitates containing melanoidin; and
d) a second separation step in which the formed precipitates are separated from the acidic mixture.

The invention further relates to melanoidin nanoparticles and melanoidin nanofibers obtained from coffee grounds.

## Description

### Field of the invention

The invention relates to nanoparticles and nanofibers containing coffee melanoidins. The invention further relates to methods for the production of coffee melanoidin nanoparticles and nanofibers.

### Background of the invention

Melanoidins are a class of brown, hydrophilic nitrogen-containing polymers which are formed during the thermal processing of foods - such as coffee, cocoa, bread, malt, barley, Brewers Spent Grain (BSG), soy, meat, and honey. During thermal processing of such foods, amino acids and reducing sugars, such as aldose and d-xylose, react to form what are termed initial Maillard reaction products. Under continued heating, melanoidins are formed by cyclizations, dehydrations, retro-aldolizations, rearrangements, isomerizations and condensations of those initial Maillard reaction products. The complexity of the Maillard reaction pathways results in a range of final reaction products, with inter alia the time of heating, type of heating, temperature, initial chemical composition of the system, moisture content, water activity and pH value being determinative of the final composition.

By way of example, the roasting process of the green coffee bean may be represented as a two-stage transformation: i) the evaporation of free water from the bean; and, ii) pyrolysis within the beans, with concomitant swelling and darkening of the beans and loss of dry weight. Oligosaccharides, polysaccharides and proteins present in the green coffee beans degrade and may then participate in the Maillard reactions. There is consequently a significant change in the chemical composition of the beans during roasting with melanoidins being a predominant product but caffeine and chlorogenic acids also being present in the final roasted coffee bean.

The chemical composition of coffee also depends on the variety. The main components of roasted beans are carbohydrates (about 38 to 42 wt.-%), melanoidins (about 25 to 28 wt.-%), lipids (about 11 to 17 wt.-%) and proteins (about 8 wt.-%). Minor components include minerals, chlorogenic acids, aliphatic acids, caffeine and trigonelline.

Melanoidins and their potential use in food, medical or technical applications have received some attention in recent years. For example, in the article by Ji Yup Kim et al.: "Coffee melanoidin-based multipurpose film formation: application to single-cell nanoencapsulation" (ChemNanoMat 10.1002/cnma.202000004) melanoidin extracted from soluble coffee powder is utilized as a material-independent, multipurpose coating material. Instantaneous complexation of the coffee melanoidin with ferric ion (Fe3+) leads to surface-adhesive aggregates, inducing sequential film deposition. The authors further disclosed a coffee melanoidin-based coating applied to single-cell nanoencapsulations of Saccharomyces cerevisiae.

So far, academia and industry have focused on synthesised melanoidins, mainly based on the Maillard reaction of glucose with the amino acid glycine. The potential of obtaining nano-scale melanoidins from other sources has not been explored so far.

### Object of the invention

It is an object of the present invention to provide a process for obtaining melanoidins in the nano scale. It is a further object of the invention to provide melanoidin products useable for a multitude of applications, the nano-scale melanoidins obtainable by an environmentally friendly and efficient process.

### Solution according to the invention

This problem is solved by providing a process for preparing a melanoidin product according to claim 1 and by providing melanoidin products according to claims 8 to 11. Advantageous embodiments and applications of the subjects of the invention are presented in claims 2 to 7 and 12 to 15.

### Definitions

In the following, any reference to one (including the articles "a" and "the"), two or another number of objects is, provided nothing else is expressly mentioned, meant to be understood as not excluding the presence of further such objects in the invention.

The terms "comprising", "comprises" and "comprised of" as used herein are synonymous with "including", "includes", "containing" or "contains", and are inclusive or open-ended and do not exclude additional, non-recited members, elements or method steps. If used, the phrase "consisting of' is closed, and excludes all additional elements. Further, the phrase "consisting essentially of' excludes additional material elements, but allows the inclusions of non-material elements that do not substantially change the nature of the invention.

When amounts, concentrations, dimensions and other parameters are expressed in the form of a range, a preferable range, an upper limit value, a lower limit value or preferable upper and limit values, it should be understood that any ranges obtainable by combining any upper limit or preferable value with any lower limit or preferable value are also specifically disclosed, irrespective of whether the obtained ranges are clearly mentioned in the context.

Further, in accordance with standard understanding, a weight range represented as being "from 0 to x" specifically includes 0 wt.-%: the ingredient defined by said range may be absent from the composition or may be present in the composition in an amount up to x wt.-%.

The terms "percent by weight", "% by weight" and "wt.-%" are used interchangeably.

The words "preferable", "preferred", "preferably", "particularly" and "desirably" are used frequently herein to refer to embodiments of the disclosure that may afford particular benefits, under certain circumstances. However, the recitation of one or more preferable, preferred, particular or desirable embodiments does not imply that other embodiments are not useful and is not intended to exclude those other embodiments from the scope of the disclosure.

As used throughout this application, the word "may" is used in a permissive sense - that is meaning to have the potential to - rather than in the mandatory sense.

As used herein, room temperature is 23°C plus or minus 2°C.

As used herein, number average molecular weight (Mn) and weight average molecular weight (Mw) are determined by gel permeation chromatography (GPC) with tetrahydrofuran (THF) as the eluent in accordance with DIN 556721:2007-08.

The term "micronized" when used in reference to a foodstuff, means a powder having a particle size of from 1 to 1500 microns, typically from 100 to 1250 microns. The term "micronized" is intended to refer to both: particles which have been produced through finely dividing materials which are originally presented in bulk form; and particles obtained by other mechanical, chemical or physical methods, including formation in solution with or without a seeding and comminution by one or more of pulverization, milling, grinding, homogenization and sonication.

The terms "micrometres", "microns" and "µm" are used interchangeably.

Grinding can, for instance, be effected using a planetary ball mill having a grinding chamber that includes a rotor shaft that is used to rotate grinding media. Reference may be made to: Le Caer et al. Mechanical Alloying and HighEnergy Ball-Milling: Technical Simplicity and Physical Complexity for the Synthesis of New Materials www.ademe.fr/recherche/manifestations/materiaux-2002/.

Milling may be performed in any high-energy mill, of which examples include: centrifugal mills; planetary ball mills; jet mills, such as spinning air flow jet mill; and, fluid energy mills. Desirably, the high-energy mill should be able to impart an impact force of at least 0.5G, for example from 0.5 to 25G, to the milling media. Non-limiting examples of mills which may find utility in the present invention are disclosed in: US Patent No. 5,522,558, US Patent No. 5,232,169, US Patent No. 6,126,097, and US Patent No. 6,145,765. Moreover, it should be noted that the present invention does not preclude milling being conducted under heat - such as described in WO 00/56486 A1 - and / or in the presence of additives, such as lubricants, surfactants, dispersants and solvents.

As used herein, by "d₅₀ particle size" is meant that the particle size distribution is such that at least 50% by weight of the particles have a particle size diameter of less than the specified value. Unless otherwise stated, that particle size is determined by dynamic light scattering. Viscosities of the compositions described herein are, unless otherwise stipulated, measured in accordance with ASTM D3236 using the Brookfield Viscometer at standard conditions of 25°C and 50% Relative Humidity (RH). The method of calibration, the spindle type and rotation speed of the Brookfield Viscometer are chosen according to the instructions of the manufacturer as appropriate for the composition to be measured.

### Process for preparing a melanoidin product

In a first aspect of the invention, the problem is solved by providing a process for preparing a melanoidin product, the process comprising:
- an extraction step in which coffee grounds are treated with an extraction agent with a pH value greater than 7 to extract a melanoidin-containing solute in a fluid phase of the extraction agent;
- a first separation step in which the fluid phase is separated from the coffee grounds;
- a precipitation step in which the fluid phase is contacted (i) with an acid to obtain an acidic mixture with a pH value lower than 4 or (ii) with an organic phase separation agent and with an acid to obtain a mixture with a pH value in the range of from 4 to 8, thereby forming precipitates containing melanoidin; and
- a second separation step in which the formed precipitates are separated from the acidic mixture.

In the context of the present invention, "coffee grounds" are fresh coffee grounds or coffee grounds left over after brewing coffee, the latter also being termed "spent coffee grounds". With the present invention, it can be exploited that spent coffee grounds can still contain significant amounts of melanoidins. Spent coffee grounds usually accumulate during the preparation of coffee. In hotels and restaurants, large quantities of spent coffee grounds are regularly disposed of as waste. Advantageously, with the present invention, valuable raw material can be extracted from the otherwise disposed spent coffee grounds.

In general, other melanoidin-containing foodstuffs like cocoa, bread, malt, barley, Brewers Spent Grain (BSG), soy, meat, or honey could be used as well for the preparation of a melanoidin product according to the invention, However, as coffee grounds have a higher melanoidin content than other sources of foodstuff, coffee grounds are preferred. Among the coffee grounds, spent coffee grounds are particularly preferred as a source from which melanoidin is extracted.

In the context of the present invention, a solute is a substance that is present in the fluid phase of the extraction agent in dissolved and/or dispersed form.

### Extraction step

In the extraction step, the coffee grounds are treated with an extraction agent to extract a melanoidin-containing solute. The solute may be present in dissolved and/or dispersed form in the fluid phase.

Suitable extraction methods include but are not limited to: reflux extraction, pressurized liquid extraction, ultrasound assisted extraction, microwave assisted extraction, pulsed electric field extraction, and/or hydro-distillation.

Preferably, the extraction step is performed at a pressure of from 1 to 8 bars (abs). More preferably, the pressure is in the range from 2 to 5 bars (abs). In a preferred variant, the pressure is adjusted by heating the extraction agent inside a closed apparatus.

In a preferred embodiment, the extraction step comprises a pressurized liquid extraction. Preferably, pressurized liquid extraction takes place at or above a temperature of 100°C, more preferably at or above 120°C. Preferably, the temperature is below 140°C. The preferred duration of the pressurized liquid extraction is greater than 10 minutes and less than 1 hour, for example between 30 and 40 minutes. It is an achievable advantage of a liquid extraction method such as the one discussed above that high solute extraction yields can be realized.

Preferably, the extraction agent is or comprises a liquid phase, and the solute is dissolved and/or dispersed in the liquid phase. More preferably, the extraction agent or the liquid phase of the extraction agent is aqueous, and the solute is dissolved and/or dispersed in the aqueous extraction agent or the aqueous phase of the extraction agent. Using water as the extraction agent or part of the extraction agent has the advantage that the extraction step can be performed in an environmentally favourable way. Furthermore, it was found that using water in the extraction step yields high melanoidin recovery rates.

Suitable extraction agents include but are not limited to an alkaline solution comprising at least one of: sodium hydroxide, potassium hydroxide, calcium hydroxide, sodium carbonate, sodium hydrogen carbonate, potassium carbonate, potassium hydrogen carbonate and ammonia (NH_{3(aq)}). The extraction agent may comprise one or more water-miscible solvents. If the water-miscible solvent(s) do not constitute more than 20 wt.-% of the extraction agent, the extraction agent is considered "aqueous" within the meaning of the present invention. Preferably, the extraction agent is aqueous and comprises at least sodium hydroxide or potassium hydroxide. More preferably, the aqueous extraction agent has the following composition: from 1 to 5 wt.-%, more preferably from 2 to 4 wt.-%, in particular 3 wt.-% sodium hydroxide or potassium hydroxide and the rest is water.

Advantages of using an aqueous extraction agent include a high dissociation constant, a low dynamic viscosity and a good diffusivity into the solid matrix of the coffee grounds, a good wettability of the solute, and short extraction times due to a high solubility of solutes from coffee grounds.

The extraction agent has a pH value greater than 7, preferably greater than 8, more preferably greater than 10. Preferably, the pH value is 14 or below, for example between 12 and 14.

### First separation step

In the first separation step, the fluid phase, which comprises the solute, is separated from the coffee grounds. Suitable separation methods include but are not limited to: centrifugation, maceration, percolation, filtering, and/or decoction.

In a suitable centrifugation method, the centrifugal force is larger than 100 G (gravitational force equivalent), preferably more than 500 G. The centrifugal force preferably is smaller than 10000 G, more preferably smaller than 5000 G, for example between 600 G and 800 G. Preferably, centrifugation takes place at a temperature above 0°C, more preferably above 5°C. Preferably, the temperature is below 50°C, more preferably below 30°C, for example between 10 and 20°C. The preferred duration of the centrifugation is greater than 1 minute, preferably greater than 5 minutes. The duration preferably is less than 4 hour, preferably less than 1 hour, for example between 10 and 30 minutes. Preferably, when the centrifugation has ended, the supernatants containing the melanoidins is collected.

### Precipitation step

In a first variant of the precipitation step the fluid phase from the first separation step is contacted with an acid to obtain an acidic mixture with a pH value lower than 4, thereby forming precipitates containing melanoidin. Preferably, the pH value is larger than 1 and smaller than 4, for example between 2 and 3.5.

Suitable acids include but are not limited to: hydrochloric acid, sulphuric acid, nitric acid, phosphoric acid and methanesulfonic acid. Preferably, the acid used in the precipitation step is hydrochloric acid.

In a second variant of the precipitation step the fluid phase is contacted with an organic phase separation agent and with an acid to obtain a mixture with a pH value in the range of from 4 to 8, thereby forming precipitates containing melanoidin.

The mixture has a pH value greater than or equal to 4. Preferably, the pH value is above 5, more preferably above 6. The mixture has a pH value lower than or equal to 8. Preferably, the pH value is lower than 8, for example between 6.5 and 7.5.

Suitable organic phase separation agents include but are not limited to: ethanol, ethyl acetate, tetrahydrofuran and 2-methyltetrahydrofuran. Preferably, the organic phase separation agent is ethyl acetate.

The weight ratio of the organic phase separation agent and the fluid phase is preferably lower than 1:3. The weight ratio of the organic phase separation agent and the fluid phase is preferably higher than 1:5, for example from 1:3.5 to 1:4.5.

Suitable acids for the second variant of the precipitation step include but are not limited to: hydrochloric acid, sulphuric acid, nitric acid, phosphoric acid and methanesulfonic acid. Preferably, the acid used in the second variant of the precipitation step is hydrochloric acid.

Preferably, the precipitation is performed at a temperature below 50°C, more preferably below 40°C. Preferably, the precipitation is performed at a temperature above 20°C.

### Second separation step

In the second separation step the formed precipitates are separated from the acidic mixture. Suitable separation methods include but are not limited to: centrifugation, maceration, percolation, filtering, and/or decoction.

The process for preparing a melanoidin product according to the invention is environmentally friendly and yields high-quality melanoidin products from coffee grounds. The melanoidin products obtained by the process according to the invention can be already in the nanoscale or can easily be transformed to nanoscale products.

### Fractionation step

In a preferred embodiment of the process for preparing a melanoidin product, the second separation step comprises or is followed by a fractionation step. In the fractionation step the melanoidin-containing precipitates are separated by means of fractionating by molecular weight.

In a preferred embodiment of the process, the fractionation is effected by ultra-filtration, preferably using requisite molecular weight cut-off membranes. Advantageously, ultra-filtration can yield a plurality of retentates which may then be further processed.

In a preferred embodiment of the process, the fractionation is effected by centrifugation or size exclusion chromatography. It is an achievable advantage of this embodiment of the invention that the appropriate selection of the minimum molecular weight of the compounds to be fractionated can substantially eliminate caffeine (molecular weight of 194 daltons) from the obtained retentates.

### Liquid phase removal step

The products of the second separation step or the fractionation step, such as the precipitates or the retentates, may be subjected to a liquid phase removal step to yield the melanoidin fractions in solid form. In case of an aqueous extraction agent, suitable methods of dehydration include but are not limited to: freeze drying, spray drying, convective drying, radiation drying, and vacuum drying. Preferably, the water removal step yields the melanoidin product as a powder. The particles of a preferred powder have an average particle size (measured as the Mass-median-diameter D₅₀) of 1 nm (nanometres) or more, more preferably 10 nm or more. Preferably, the particles of the powder have an average particle size (D₅₀) of 50 µm (micrometres) or less, more preferably 5 µm or less, for example between 10 nm and 1 µm.

### Nanoparticle production step / nanofiber production step

The products of the second separation step, the fractionation step or the liquid phase removal step, such as the precipitates or the retentates, may be subjected to a nanoparticle production step in which nanoparticles are produced. Preferably, the nanoparticle production step comprises at least one method selected from the group of electrospinning, laser ablation, freeze drying, spray drying, convective drying, radiation drying, and vacuum drying.

The products of the second separation step, the fractionation step or the liquid phase removal step, such as the precipitates or the retentates, may be subjected to a nanofiber production step in which nanofibers are produced. Preferably, the nanofiber production step comprises electrospinning. The nanofiber production step may include the addition of one or more auxiliary components to the melanoidin fraction. These auxiliary components may improve the processability or the formation of fibers. In a preferred embodiment polyvinyl alcohol is added as an auxiliary component.

In one preferred embodiment the second separation step comprises or is followed by a fractionation step in which a low-molecular weight fraction of the formed precipitates with an average molecular weight of less than 10 kDa is obtained, and wherein the second separation step is followed by a nanoparticle production step in which nanoparticles are produced from the low-molecular weight fraction. Preferably, the nanoparticle production step comprises at least one method selected from the group of electrospinning, laser ablation, freeze drying, spray drying, convective drying, radiation drying, and vacuum drying.

In another preferred embodiment the second separation step comprises or is followed by a fractionation step in which a high-molecular weight fraction of the formed precipitates with an average molecular weight of at least 10 kDa is obtained, and wherein the second separation step is followed by a nanofiber production step in which nanofibers are produced from the high-molecular weight fraction. Preferably, the nanofiber production step comprises electrospinning. The nanofiber production step may include the addition of one or more auxiliary components to the melanoidin fraction. These auxiliary components may improve the processability or the formation of fibers. In a preferred embodiment polyvinyl alcohol is added as an auxiliary component.

### Pre-treatment

In another preferred embodiment of the process for preparing a melanoidin product, the extraction step is preceded by one or more pre-treatment steps.

A preferred pre-treatment is a micronization of the coffee grounds. In the context of the present invention, "micronization" is a process of reducing the average diameter of the particles of the coffee grounds. Preferably, the micronization is performed by milling or grinding. A preferred mill is a ball mill. The average diameter of the coffee grounds after micronization preferably is below 1500 µm, preferably below 1250 µm. It preferably is above 1 µm, more preferably above 100 µm.

Another preferred pre-treatment is drying of the coffee grounds. Preferably, during drying the coffee grounds are exposed to a temperature above 50°C, more preferably above 70°C. Preferably, the drying temperature is below 110°C, for example between 70°C and 90°C. The water content of the coffee grounds after drying preferably is below 5 %, more preferably below 2 %. The preferred duration of the drying is greater than 1 hour and less than 2 hours.

Another preferred pre-treatment is a defatting of the coffee grounds. In a preferred defatting method, at least a portion of the lipids and/or oils present in the coffee grounds are extracted using a suitable solvent, for example carbon dioxide. Preferably, the carbon dioxide is applied at a pressure and temperature such that it is a supercritical fluid.

In another preferred defatting method, which is especially suited for spent coffee grounds, at least a portion of the lipids and/or oils present in the coffee grounds are extracted using an organic solvent, for example ethanol, diethyl ether or chloroform.

The lipid content of the coffee grounds after defatting preferably is below 2 %, more preferably below 1 %. With the defatting, it can advantageously be achieved that the yield of the subsequent extraction step is increased and that the potential formation of oil-extracting solvent emulsions is prevented.

The above pre-treatments and other pre-treatments can be performed alone or in any combination. Preferably, micronization is performed before drying and/or defatting rather than after the latter step(s).

### Melanoidin nanoparticles and nanofibers

In a second aspect of the invention, the problem is solved by providing melanoidin nanoparticles and melanoidin nanofibers obtained by a process according to the invention.

In one preferred embodiment the melanoidin product is a nanoparticle.

Preferably, the nanoparticle has an average diameter larger than 1 nm, more preferably larger than 10 nm. Preferably, the nanoparticle has an average diameter smaller than 1000 nm, more preferably smaller than 800 nm, for example between 40 nm and 600 nm

Preferably, the melanoidin nanoparticle comprises more than 70% by weight of melanoidins, more preferably more than 80% by weight of melanoidins, even more preferably more than 90% by weight of melanoidins, in particular more than 95% by weight of melanoidins.

It is further preferred that the melanoidins comprise from 1 % by weight to 15 % by weight of phenolic compounds. Preferably, the melanoidins comprise more than 2 % by weight of phenolic compounds, more preferably more than 3 % by weight of phenolic compounds. Preferably, the melanoidins comprise less than 14 % by weight of phenolic compounds, more preferably less than 13 % by weight of phenolic compounds, for example from 4 to 12 % by weight of phenolic compounds.

The term "phenolic compounds" means the total of all phenols and polyphenols that are present in the melanoidin products, including but not limited to phenol, polyphenols and phenolic acids like gallic acids, chlorogenic acids, caffeic acids, salicylic acids.

Quantitative analysis of the phenolic compounds can be performed using the Folin Ciocalteu reagent which is well established in the art. The Folin Ciocalteu reagent is a mixture of phosphomolybdate and phosphotungstate and is typically used for the colorimetric in vitro assay of phenolic and polyphenolic antioxidants.

It is further preferred that the melanoidins comprise from 1 % by weight to 40 % by weight of proteins. Preferably, the melanoidins comprise more than 5 % by weight of proteins, more preferably more than 10 % by weight of proteins. Preferably, the melanoidins comprise less than 35 % by weight of proteins, for example from 20 to 30 % by weight of proteins.

Quantitative analysis of the protein content can be performed using the Bradford protein assay which is a spectroscopic analytical procedure used to measure the concentration of protein in a solution and which is well established in the art.

In another preferred embodiment the melanoidin product is a nanofiber.

Preferably, the nanofiber has an average diameter larger than 1 nm, more preferably larger than 10 nm. Preferably, the nanofiber has an average diameter smaller than 1000 nm, more preferably smaller than 800 nm, for example between 40 nm and 600 nm.

Preferably, the melanoidin nanofiber comprises more than 50% by weight of melanoidins, more preferably more than 70% by weight of melanoidins, even more preferably more than 90% by weight of melanoidins, in particular more than 95% by weight of melanoidins.

It is further preferred that the melanoidins comprise from 1 % by weight to 15 % by weight of phenolic compounds. Preferably, the melanoidins comprise more than 2 % by weight of phenolic compounds, more preferably more than 3 % by weight of phenolic compounds. Preferably, the melanoidins comprise less than 14 % by weight of phenolic compounds, more preferably less than 13 % by weight of phenolic compounds, for example from 4 to 12 % by weight of phenolic compounds.

It is further preferred that the melanoidins comprise from 1 % by weight to 40 % by weight of proteins. Preferably, the melanoidins comprise more than 5 % by weight of proteins, more preferably more than 10 % by weight of proteins. Preferably, the melanoidins comprise less than 35 % by weight of proteins, for example from 20 to 30 % by weight of proteins.

It has been found that melanoidin nanoparticles and melanoidin nanofibers obtained from coffee grounds according to the invention have advantageous properties that enable a broad range of applications. These advantageous properties include, but are not limited to, a good solubility in water and other solvents, a broad light absorption range as well as antimicrobial and antioxidant properties.

Furthermore, it has been found that melanoidin nanoparticles and melanoidin nanofibers obtained from coffee grounds according to the invention have a high thermal conversion efficiency and superior electrical conductivity compared to known melanoidin products.

### Uses of melanoidin nanoparticles and nanofibers

A further aspect of the invention is an electronic component comprising a melanoidin product according to the invention. The melanoidin product may comprise melanoidin nanoparticles and/or melanoidin nanofibers.

The electrical conductivity of the nano-scale melanoidin products according to the invention enables the development and provision of environmentally friendly and sustainable electronic components. Examples for electronic components include but are not limited to: computers, memory storages, displays, optoelectronic devices, printed circuits, conductive liquids, solar collectors, solar heating, solar evaporation.

A further aspect of the invention is a conductive liquid comprising a melanoidin product according to the invention. The melanoidin product may comprise melanoidin nanoparticles and/or melanoidin nanofibers.

In a preferred embodiment the conductive liquid is a conductive ink. The conductive ink according to the invention can be used in a variety of applications in printed electronics, for example for flexible circuits, resistors, heating elements, displays, sensors, RFID antenna, smart tattoos.

A further aspect of the invention is the use of a melanoidin product according to the invention for a photothermal application, in particular photothermal therapy, photodynamic therapy, photothermal catalysis, photothermal antibacterial treatment, solar collectors, solar heating, solar evaporation. The melanoidin product may comprise melanoidin nanoparticles and/or melanoidin nanofibers.

A further aspect of the invention is the use of a melanoidin product according to the invention for drug delivery applications. The melanoidin product may comprise melanoidin nanoparticles and/or melanoidin nanofibers.

In a preferred embodiment the melanoidin product is used for topical drug delivery. In another preferred embodiment the melanoidin product is used for transdermal drug delivery. In both embodiments a controlled release of drugs or other active components can be achieved. Due to its favourable biocompatibility the melanoidin product is able to degrade through the natural pathways into the body.

Preferably, melanoidin nanoparticles and/or nanofibers according to the invention are used to provide a matrix in which a drug or an active component is located. When applied to the skin, for example as a cream, lotion, ointment or an adhesive tape, the melanoidin nanoparticles and/or nanofibers are able to penetrate the skin and to deliver the drug or active component to the body.

In a further embodiment for drug delivery the melanoidin product is used in a medical product for hypodermic applications. In a favourable medical product of this type the melanoidin product is combined with a drug or active component. The medical product is placed under the skin, for example as a wound tissue after a surgery or as a depot of drugs that continuously delivers amounts of drugs into the body for a controlled medical treatment over a certain time period.

In a further embodiment for drug delivery the melanoidin product is used in a medical product for oral applications. In a favourable medical product of this type the drug or active component is foreseen to be delivered to the gut. Using the melanoidin product as a carrier the drug or active component is able to penetrate the intestine wall in this case.

Melanoidin nanoparticles and nanofibers according to the invention are especially well suited for medical applications due to their good biocompatibility and beneficial properties, for example antimicrobial properties.

A further aspect of the invention is the use of a melanoidin product according to the invention for bio-electronics applications. The melanoidin product may comprise melanoidin nanoparticles and/or melanoidin nanofibers. Due to their electrically conductive properties melanoidin products according to the invention are especially well suited for these applications.

In one variant the melanoidin product is used for neural bio-interfacing applications, in particular for implants. Examples include but are not limited to bio-electrodes, neural interfaces, neural electrode-tissue interfaces, cardiac interfaces, bone tissue biomaterials, artificial tissue biomaterials, tissue regeneration, stretchable bio-electronics, conductive hydrogels for neural interfaces.

In a further variant the melanoidin product is used for bio-actuator applications, for example for neural stimulation, mechanical actuation, bio-actuators, dynamic actuation like e-skin, e-muscle applications, prostheses.

For all medical applications the terms "skin" and "body" may refer to human skin and body as well as to animal skin and body.

A further field of application of the inventive melanoidin nanoparticles and melanoidin nanofibers obtained from coffee grounds are cosmetic compositions. Further subjects of the invention therefore include but are not limited to the following list of cosmetic embodiments, wherein the term "nanoscale melanoidin product" means melanoidin nanoparticles having an average diameter of 1 nm to 1000 nm and/or melanoidin nanofibers having an average diameter of 1 nm to 1000 nm.

The term "cosmetic" is used herein in accordance with the definition applied in US Federal Food, Drug and Cosmetic Act - as Amended Through P.L. 107-377, 19th December 2002 - as "articles intended to be rubbed, poured, sprinkled, or sprayed on, introduced into, or otherwise applied to the human body...for cleansing, beautifying, promoting attractiveness, or altering the appearance". In its broadest disclosure, the form of the cosmetic composition of the present invention is not particularly limited and may be a liquid, emulsion, paste, gel, solid or compact powder. Particular examples of the cosmetic composition include: facial cleansers; facial treatments such as skin lotion, skin milk, cream, gel, serum and facial masks; facial and eye make-up preparations, such as face powder, foundation, lip rouge, blush, eyeliner, mascara, eye shadow and eyebrow pencil; tattoos, including permanent makeup; hair cosmetic compositions such as shampoo, rinse, conditioner, hair styling agent, hair treatment and hair colorants. Included in particular within this definition are: eye and facial makeup preparations; tattoos, including permanent makeup; and, hair colours.

The term "solid" as used herein with respect to cosmetic compositions references the state of the composition at room temperature and at atmospheric pressure: the composition possesses a high consistency which conserves its form during storage and does not flow under its own weight.

The term "structurant", as used herein, means any material known or otherwise effective in providing suspending, gelling, viscosifying, solidifying and/or thickening properties to the composition, or those materials which otherwise provide structure to the final product form. These solid structurants include one or more solid crystalline or other non-polymeric suspending agents suitable for topical application to human skin. Suitable suspending agents are those that can form in the composition a crystalline or other matrix within which volatile solvents, non-volatile solvents, or other liquid components of the composition are contained. Such materials will typically be solids under ambient conditions and include organic solids, waxes, crystalline or other gellants, or combinations thereof. A structurant provides a uniform distribution of the particulate active throughout the product and also controls product hardness or rheology. For completeness, crystalline stucturants suitable for use in the solid compositions disclosed herein are described inter alia in US Patent No. No. 5,552,136, (Motley), US Patent No. 5,976,514 (Guskey et al.), and US Patent No. No. 5,891,424 (Bretzler et al.).

The term "compact powder" refers to a mass of product whose cohesion is at least partly provided by compacting or pressing during manufacture. In particular, by taking a measurement using a TA.XT.plus Texture Analyser texturometer - available from Stable Micro Systems - the compact powder should have a pressure resistance of between 0.1 and 2.5 kg relative to the surface area of the spindle used. The measurement of this resistance is performed by moving an SMS P/3 flat-headed cylindrical spindle (7.07 mm²) over a distance of 1.5 mm and at a speed of 0.5 mm/s.

The term "tattoo" references an indelible mark or figure on the mammalian body obtained through the insertion a pigment under the skin using needles, scalpels, or other related equipment. The term is intended to encompass both intra-epidermal tattoos and intra-dermal tattoos which will be distinguished inter alia by their degree of permanence.

As used herein, "keratinous tissue" refers to the keratin-containing layers disposed as the outermost protective covering of mammals which include, but are not limited to, skin, hair, nails, and cuticles. There is no particular intention to limit the locus of the keratinous tissue to which the present cosmetic compositions may be applied: mention may be made of keratinous tissue disclosed on the face, neck, chest, back, arms (including underarms), hands, legs, feet, buttocks and scalp.

The term "epidermis" as used herein refers to the outer layer of skin of which the predominant cell type is the keratinocyte. As would be understood by the skilled artisan, the epidemis is conventionally divided into five strata: stratum corneum; stratum granulosum; stratum spinosum; and stratum basale. The stratum corneum is often referenced as being non-viable and contains many layers of dead, anucleated keratinocytes that are essentially filled with keratin. The stratum lucidum contains two to three layers of anucleated cells. The stratum granulosum contains two to four layers of cells that are held together by desmosomes that contain keratohyaline granules. The stratum spinosum contains eight to ten layers of modestly active dividing cells that are also held together by desmosomes. The stratum basale contains a single layer of columnar cells that actively divide by mitosis and provide the cells that are destined to migrate through the upper epidermal layers to the stratum corneum.

The term "dermis" as used herein refers to the layer of skin between the epidermis and the subcutaneous tissues. The dermis provides structural support to the skin and comprises non-cellular collagen fibers derived from fibroblasts.

The term "hair" herein means mammalian keratin fibers and includes scalp hair, facial hair and body hair. In addition, the term encompasses hair both hair which is attached to a living subject and also hair that has been removed there from. For completeness, a "hair shaft" or "hair fiber" means an individual hair strand.

The expression "proximal to the scalp" means that portion of an extended, or substantially straightened, hair shaft that is closer in distance to the scalp than to the end of the hair. Thereby 50% of the hair fiber length would be considered proximal to the scalp and 50% of the hair fiber length would be distal to the scalp. When used "x cm" proximal to the scalp means a distance "x" along an extended or substantially straightened hair measured from the scalp as the endpoint.

"Cosmetically acceptable" means that the compositions, formulations or components described herein are suitable for use in contact with human keratinous tissue without undue toxicity, incompatibility, instability, allergic response, and the like. All compositions and formulations described herein which have the purpose of being directly applied to keratinous tissue are limited to those being cosmetically acceptable.

The term "topical application", as used herein, means to apply or spread the compositions of the present invention onto the surface of the skin.

Water, for use as a (co-)solvent herein, is intended to mean water of low solids content as would be understood by a person of ordinary skill in the art. The water may, for instance, be distilled water, demineralized water, deionized water, reverse osmosis water, boiler condensate water, or ultra-filtration water. Tap water may be tolerated in certain circumstances but is not tolerated within the tattoo ink composition described herein.

As used herein "solvents" are substances capable of dissolving another substance to form a uniform solution; during dissolution neither the solvent nor the dissolved substance undergoes a chemical change. Solvents may either be polar or non-polar. The term "alcoholic solvent" encompasses such solvents which are any water-soluble monoalcohols, diols or polyols that are liquids at 25°C at atmospheric pressure.

If an organic liquid product has a boiling point region, then the onset (the lowest temperature) of the boiling point range at atmospheric pressure is taken as the nominal boiling point. Where necessary any measurement of the initial boiling point for materials should be conducted in accordance with ASTM Standard Test Method D1078-95, or its most current version.

The term "water-miscible organic solvent", as used herein, refers to an organic solvent that is completely miscible with water at room temperature. In this regard, a particular preference may be noted for organic solvents which are soluble, freely soluble or very soluble in water and are thereby characterized by requiring ≤ 30 ml of water to dissolve 1 g of organic solvent at room temperature (https://www.sigmaaldrich.com/united-kingdom/technical-services/solubility.html).

The term "water-immiscible organic solvent", as used herein refers to organic solvents that form a two-phase system with water. In this regard, a particular preference may be noted for organic solvents which are slightly soluble, very slightly soluble or practically insoluble in water and are thereby characterized by requiring ≥ 100 ml of water to dissolve 1 g of organic solvent at room temperature (https://www.sigmaaldrich.com/united-kingdom/technical-services/solubility.html).

As used herein, the term "dispersion" refers to a composition that contains discrete particles that are distributed throughout a continuous liquid medium.

As used herein, the term "surface tension" refers to the force required to increase the unit area of a surface of a liquid or of an interface between two liquids or between a liquid and a gas, generally stated in units of dynes/cm. Surface tensions described herein are measured by the Du Noüy ring method utilizing an EasyDyne tensiometer model K20 marketed by Krüss USA, Matthews, N.C.

As used herein, "C₁-Cₙ alkyl" group refers to a monovalent group that contains 1 to n carbons atoms, that is a radical of an alkane and includes straight-chain and branched organic groups. As such, a "C₁-C₆ alkyl" group refers to a monovalent group that contains from 1 to 6 carbons atoms, that is a radical of an alkane and includes straight-chain and branched organic groups. Examples of alkyl groups include, but are not limited to: methyl, ethyl, propyl, isopropyl, n-butyl, isobutyl, sec-butyl, and tert-butyl. In the present invention, such alkyl groups may be unsubstituted or may be substituted with one or more halogen. Where applicable, a preference for a given substituent will be noted in the specification.

The term "C₂-C₆ alkylene" as used herein, is defined as saturated, divalent hydrocarbon radical having from 2 to 6 carbon atoms.

The present compositions are defined herein as being "substantially free" of certain compounds, elements, ions or other like components. The term "substantially free" is intended to mean that the compound, element, ion or other like component is not deliberately added to the composition and is present, at most, in only trace amounts which will have no (adverse) affect on the desired properties of the composition. An exemplary trace amount is less than 1000 ppm by weight of the composition. The term "substantially free" encompasses those embodiments where the specified compound, element, ion, or other like component is completely absent from the composition or is not present in any amount measurable by techniques generally used in the art.
Cosmetic embodiment 1
   is a cosmetic composition comprising a nanoscale melanoidin product.
Cosmetic embodiment 2
   is a cosmetic composition comprising a nanoscale melanoidin product, wherein the nanoscale melanoidin product has a weight average molecular weight of from 200 daltons (Da) to 300 kilodaltons (kDa), preferably from 1 to 300 kDa, more preferably from 1 to 100 kDa, most preferably from 10 to 100 kDa.
Cosmetic embodiment 3
   is a cosmetic composition comprising a nanoscale melanoidin product which possess a more narrowly defined molecular weight, such as from 10 to 30 kDa, from 30 to 50 kDa, from 50 to 100 kDa or from 100 to 200 kDa where, for example, a particular pigmentary or light absorption effect is desired. It is also envisaged that more than one nanoscale melanoidin product fraction may be present: for example, a first fraction based on a nanoscale melanoidin product possessing a weight average molecular weight of from 10 to 30 kDa may be combined in the cosmetic composition with a second fraction based on a nanoscale melanoidin product having a weight average molecular weight of from 50 to 100 kDa or from 100 to 200 kDa.
Cosmetic embodiment 4
   is a cosmetic composition comprising a nanoscale melanoidin product, the cosmetic composition being non-powdered solid cosmetic composition, a compact powder cosmetic composition or an aqueous liquid cosmetic composition.
Cosmetic embodiment 5
   is a cosmetic composition comprising a nanoscale melanoidin product that is essentially free of caffeine.
Cosmetic embodiment 6
   is an aqueous hair dyeing composition comprising:
   a) a nanoscale melanoidin product, wherein said nanoscale melanoidin product has a weight average molecular weight of from 200 daltons to 300kDa, preferably from 1 to 300 kDa and more preferably from 1 to 100 kDa; and
   b) at least one auxiliary agent selected from the group consisting of: wetting agents, swelling agents, penetrants, pH regulators, surfactants, thickeners, antimicrobial agents, and perfumes.
Cosmetic embodiment 7
   is an aqueous hair dyeing composition comprising, based on the weight of the composition:
   a) from 5 to 20 wt.-% of a nanoscale melanoidin product, said nanoscale melanoidin product having a weight average molecular weight of from 200 daltons to 300 kDa, preferably from 1 to 300 kDa and more preferably from 1 to 100 kDa; and
   b) from 5 to 40 wt.-% of at least one auxiliary agent selected from the group consisting of: wetting agents, swelling agents, penetrants, pH regulators, surfactants, thickeners, antimicrobial agents, and perfumes; and
   c) from 40 to 90 wt.-% of water.

As regards the aqueous hair dye compositions, it is preferred that said compositions contain from 40 to 90 wt.-%, preferably from 40 to 80 wt.-% and more preferably from 55 to 75 wt.-%, based on the weight of the composition, of water, the sum of components a), b) and c) above always being 100 wt.-%. In an alternative but not mutually exclusive characterization, the aqueous hair dye composition may be defined by a viscosity of from 0.005 to 50 Pa.s, as measured using a Brookfield viscometer at 25°C.

Component b) in cosmetic embodiment 7 represents categorizations based on the benefit provided by the agent or the agent's postulated mode of action. This categorization is made for convenience: a given chemical ingredient - such as ammonia for example - might provide more than one benefit or operate via more than one mode of action.

Exemplary wetting agents, which may be used alone or in combination, include but are not limited to: glycerin; propylene glycol; sorbitol; 1,3-butylene glycol; polyvinylpyrollidone (PVP); polyethylene glycols (PEG); polypropylene glycol (PPG); PEG/PPG-block copolymers; and, PEG/PPG-random copolymers. An illustrative PEG/PPG-random copolymer is PEG/PPG - 8/17.

Exemplary swelling agents include ammonia and monoethanolamine (MEA). The ammonia, when included, would be present in the aqueous compositions of the present invention as an ammonia solution NH₃(aq) which encompasses weakly basic solutions of ammonia in water which may referred to in the art as ammonium hydroxide, ammonia water, ammonia liquor, aqua ammonia, aqueous ammonia, or simply ammonia. While the term "ammonium hydroxide" suggests a base with the composition [NH₄⁺][OH⁻], it is virtually impossible to isolate samples of NH₄OH, insomuch as these ions do not comprise a significant fraction of the total amount of ammonia in an ammonia solution, except in the case of extremely dilute ammonia solutions.

Exemplary penetrants, which may be used alone or in combination include: monohydric alcohols having C1-C6 alkyl group such as ethanol, 1-propanol, 2-propanol, 1-butanol, and 2-butanol; polyhydric alcohols having from 3 to 8 carbon atoms such as propanediol, butanediol, pentanediol, hexanediol, hexanetriol, heptanediol, heptanetriol, octanediol, octanetriol, isoprene glycol, propylene glycol, glycerin, and diethylene glycol monoethyl ether; esters of said polyhydric alcohols; N-alkylpyrrolidones which are liquid at room temperature such as N-methyl-2-pyrrolidone, N-ethyl-2-pyrrolidone, N-propyl-2-pyrrolidone, N-butyl-2-pyrrolidone, and N-cyclohexyl-2-pyrrolidone; C2-C6 alkylene carbonates such as ethylene carbonate and propylene carbonate; aromatic alcohols such as benzyl alcohol, benzyloxyethanol, cinnamyl alcohol, p-anisyl alcohol, p-methylbenzyl alcohol, phenoxyethanol, phenoxyisopropanol, 2-benzyl ethanol and β-phenylethyl alcohol. A preference for 2-propanol, 1,2-hexanediol and benzyl alcohol might be mentioned.

Examples of pH adjusters include: mineral acids, such as phosphoric acid; hydroxycarboxylic acids such as lactic acid, glycolic acid, citric acid, 3-hydroxybutyric acid, 4-hydroxybutyric acid, 2-hydroxybutanedioic acid, 2,3dihydroxybutanedioic acid, 3-hydroxyvaleric acid, 5-hydroxyvaleric acid, 6-hydroxycaproic acid and the alkali metal or alkaline earth metal salts thereof; ammonia; alkali metal hydroxides; alkali metal carbonates; alkali metal bicarbonates; alkaline earth metal hydroxides; alkaline earth metal carbonates; and, alkaline earth metal bicarbonates.

The aqueous hair dye compositions of the present invention will typically comprise up to 10 wt.-%, for example up to 5 wt.-% or up to 3 wt.-%, based on the weight of the composition, of surfactant. A wide variety of surfactants may be used herein - both for emulsification of the dispersed phase and to provide acceptable spreading of the applied composition - but desirable surfactants will be selected from the group consisting of: anionic surfactants, nonionic surfactants, amphoteric surfactants, non-lathering surfactants, emulsifiers, and mixtures thereof. US Patent No. 6,280,757 (McAtee et al.) provides an instructive disclosure on suitable surfactants.

Anionic Surfactants: Non-limiting examples of anionic surfactants useful in the compositions of the present invention are disclosed US Patent No. 3,929,678 (Laughlin et al.) and US Patent No. 4,557,853. Mention may be made of anionic surfactants selected from the group consisting of: i) fatty acid soaps based on fatty acids having from 8 to 36 or from 8 to 24 carbon atoms; ii) monoalkyl, dialkyl, and trialkylphosphate salts; iii) sarcosinates, such as sodium lauroyl sarcosinate, sodium myristoyl sarcosinate and sodium cocoyl sarcosinate; iv) sulfates, including alkyl and alkyl ether sulfates of which mention may be made of sodium lauryl sulfate, ammonium lauryl sulfate, ammonium laureth sulfate, sodium laureth sulfate, sodium trideceth sulfate, ammonium cetyl sulfate and sodium cetyl sulfate; v) isethionates such as sodium lauroyl isethionate and ammonium cocoyl isethionate; vi) taurates such as sodium lauroyl methyl taurate and sodium cocoyl methyl taurate; vii) lactylates such as sodium lauroyl lactylate, triethanolamine lauroyl lactylate and sodium caproyl lactylate; viii) glutamates such as sodium lauroyl glutamate, sodium myristoyl glutamate, and sodium cocoyl glutamate; and, mixtures thereof.

Non-Ionic Surfactants: Suitable nonionic surfactants having utility in the present disclosure include: alkyl glucosides; alkyl polyglucosides; polyhydroxy fatty acid amides; alkoxylated fatty acid esters; sucrose esters; amine oxides; and mixtures thereof. Mention may, for example, be made of: C8-C14 glucose amides, C8-C14 alkyl polyglucosides, sucrose cocoate, sucrose laurate, lauramine oxide, cocoamine oxide and mixtures thereof.

Amphoteric Surfactants: The term "amphoteric surfactant" as used herein, is also intended to encompass zwitterionic surfactants. Useful amphoteric surfactants, which may be used alone or in combination, include but are not limited to: derivatives of aliphatic secondary and tertiary amines, preferably wherein the nitrogen is in a cationic state and wherein at least one aliphatic radical contains an ionizable water solubilizing group such as a carboxyl, sulfonate, sulfate, phosphate or phosphonate group, betaines, sultaines, hydroxysultaines, alkyliminoacetates, iminodialkanoates, and aminoalkanoates.

Non-Lathering Surfactants: Given that the present hair dye compositions are not conventionally intended to have a cleansing functionality, they need not therefore be lathering systems and can comprise non-lathering surfactants. Exemplary non-lathering surfactants having utility herein include: polyethylene glycol 20 sorbitan monolaurate (Polysorbate 20); Polysorbate 60; Polysorbate 80; polyoxyethylene 20 sorbitan trioleate (Polysorbate 85); Steareth20; Ceteth-10; Ceteareth-20; cetyl phosphate; potassium cetyl phosphate; diethanolamine cetyl phosphate; glyceryl stearate; PPG-2-methyl glucose ether distearate; and, PEG-100 stearate.

Emulsifier systems: In addition to the above, the use of emulsifier(s) is not precluded in the present disclosure. Mention may be made of the following emulsifier mixtures: PROLIPID^{®} 141 (glyceryl stearate, behenyl alcohol, palmitic acid, stearic acid, lecithin, lauryl alcohol, myristyl alcohol and cetyl alcohol) available from ISP; PROLIPID^{®} 151 (Glyceryl stearate, cetearyl alcohol, stearic acid, 1-propanamium, 3-amino-N-(2-(hydroxyethyl)-N-N-Dimethyl,NC(16-18) Acyl Derivatives, Chlorides), available from ISP; POLAWAX^{®} NF (Emulsifying wax NF) available from Croda; INCROQUAT^{®} BEHENYL TMS (behentrimonium sulfate and cetearyl alcohol) available from Croda; and EMULLIUM^{®} DELTA (cetyl alcohol, glyceryl stearate, peg-75 stearate, ceteth-20 and steareth-20) available from Gattefosse.

The aqueous hair dye compositions of the present disclosure may comprise from 0 to 10 wt.-%, for example from 0 to 5 wt.-%, based on the weight of the composition, of one or more thickening agents. Said thickening agents may be selected from: i) carboxylic acid polymers, such as those members of the CARBOPOL^{®}900 series available from B.F. Goodrich; ii) acrylates/C₁₀-C₃₀ alkyl acrylate crosspolymers, such as CARBOPOL^{®}1342, CARBOPOL^{®}1382, PEMULEN^{®} TR-1 and PEMULEN^{®} TR-2 available from B.F. Goodrich; iii) crosslinked polyacrylate polymers, which may be cationic or nonionic polymer; iv) polyacrylamide polymers, in particular nonionic polyacrylamide polymers; iv) multi-block copolymers of acrylamides and substituted acrylamides with acrylic acids and substituted acrylic acids; v) polysaccharides including scleroglucans, cellulose and cellulose derivatives, such as carboxymethylcellulose and alkyl hydroxyalkyl cellulose ethers; vi) modified starches; vii) gums such as xantham gum, acacia gum, alginin, sodium alginate, locust bean gum and guar gum; viii) proteins such as collagen, albumen and gelatin; and, mixtures thereof.

The aqueous hair dye compositions of the present disclosure may comprise from 0 to 5 wt.-%, preferably from 0 to 2 wt.-%, based on the weight of the composition, of at least one antimicrobial agent. It is preferred that the or each antimicrobial agent included in the composition is a crystalline particulate that is insoluble in water. Exemplary antimicrobial agents include but are not limited to: sulfur; piroctone olamine; selenium sulfides, as described in US Patent No. 2,694,668, US Patent No. 3,152,046 and US Patent No. 4,089,945; and, pyridinethione salts, as described in US Patent No. 3,753, 196, US Patent No. 4,345,080, US Patent No. 4,323,683, and US Patent No. 4,470,982.

Examples of perfumes which may be included in the aqueous hair dyeing composition include vanillin, cinnamic alcohol, heliotropine, coumalin, 2-methyl-3-(3,4-methylenedioxyphenyl)-propanal, 4-(4-hydroxyphenyl)-2-butanone, benzaldehyde, anisyl alcohol, 3,4-dimethoxybenzaldehyde, heliotropyl acetate, phenyl acetaldehyde dimethylacetal, phenoxyethyl alcohol, phenyl acetaldehyde glycerylacetal, benzyl alcohol, phenylethyl alcohol, franeol, sugar lactone, menthol, ethyl diglycol, benzyl acetate, linalool, camphor, terpineol, citronellol, geraniol, 2,6-nonadienol, methyloctyl 20 carbonate, 3,7-dimethyl-2,6-octadienal and nonanal.

The hair dye of the present composition may be applied using conventional means to at least a portion of the hair shaft, including by finger touch, by hand, by brush or by another implement, optionally in concert with a means of delivering thermal energy or a suitable electromagnetic radiation. The composition(s) may be applied to wet hair or to 25 dry hair. The amount applied will vary, dependent upon the thickness and length of the hair, and the desired effect.

The hair dye composition(s) may be applied to substantially all of the hair or alternatively to a portion of the hair. In one embodiment, the composition may be applied to a portion of the hair that is proximal to the scalp, for example, from 0 to 10 cm or from 0 to 5 cm proximal to the scalp. This may be desirable for the user modify the color of the 30 roots of newly grown hair.
Cosmetic embodiment 8
   is an aqueous tattoo ink composition, comprising:
   a) at least one colorant, wherein said at least one colorant comprises a nanoscale melanoidin product, said nanoscale melanoidin product having a weight average molecular weight of from 200 daltons to 300 kDa, preferably from 1 to 300 kDa and more preferably from 1 to 100 kDa; and
   b) at least one auxiliary selected from the group consisting of: binders, thickeners, pH regulators, surfactants, humectants, antimicrobial agents, anti-inflammatory agents, anti-oxidants, antiseptics, and local anesthetics.
   Optionally, the aqueous tattoo ink can further comprise at least one co-solvent.
Cosmetic embodiment 9
   is an aqueous tattoo ink composition comprising, based on the weight of the composition, wherein the total composition sums up to 100 wt.-%:
   a) from 10 to 40 wt.-% of water;
   b) from 30 to 60 wt.-% of at least one colorant, wherein said at least one colorant comprises a nanoscale melanoidin product, said nanoscale melanoidin product having a weight average molecular weight of from 200 daltons to 300 kDa, preferably from 1 to 300 kDa and more preferably from 1 to 100 kDa;
   c) from 0 to 15 wt.-% of at least one co-solvent; and
   d) from 5 to 15 wt.-% of at least one auxiliary selected from the group consisting of: binders, thickeners, pH regulators, surfactants, humectants, antimicrobial agents, anti-inflammatory agents, anti-oxidants, antiseptics, and local anesthetics.

Component d) in cosmetic embodiment 9 represents categorizations based on the benefit provided by the auxiliary or the auxiliary's postulated mode of action. This categorization is made for convenience: a given chemical ingredient - such as polyethylene glycol for example - might provide more than one benefit or operate via more than one mode of action. In this regard, it is repeated here that the nanoscale melanoidin products themselves provide a pigmentary function and act as antimicrobial agents, anti-inflammatory agents and anti-oxidants and can thereby minimize the need to provide adjunct ingredients having these functionalities.

The aqueous tattoo ink composition of the present invention can include further colorants in addition to the aforementioned melanoidins. Said colorants are by necessity water-soluble or water-dispersible and may be selected from: inorganic pigments; organic pigments; natural dyes; synthetic dyes; and, combinations thereof. The use of organic, lacquered or lacquer pigments ("lake pigments") - which are obtained by precipitating a natural or synthetic dye with a metal salt - is also envisaged. It is however preferred that the formulation is substantially free of metal particles and / or is substantially free of microencapsulated colorants.

For completeness, exemplary inorganic pigments include but are not limited to: metal oxides, such as iron oxide red, iron oxide yellow, iron oxide black, anatase, brookite, rutile, aluminum oxide, zirconium oxides, cobalt oxides, cerium oxides, nickel oxide, chromium oxide, nickel-chromium oxides, zinc oxides and composite oxides; metal hydroxides, such as calcium hydroxide, iron hydroxides, aluminum hydroxide, chromium hydroxide, magnesium hydroxide and composite metal hydroxides; Prussian blue; iron sulfide; manganese violet; carbon black; mica; and, kaolin.

The aqueous tattoo ink composition may comprise at least one water-miscible organic co-solvent. When present, the water and said at least one water-miscible organic solvent should be mixed at a ratio by weight of from 20:80 to 80:20, for example from 30:70 to 70:30. In an additional statement of preference, which is not intended to be mutually exclusive of those ratios mentioned above, it is preferred that the water-miscible organic solvent or the mixture of water-miscible organic solvents included in this part are selected and added to water in an amount sufficient to reduce the surface tension of the water/solvent combination to less than 64 dynes/cm at room temperature.

The at least one water-miscible organic solvent of the present invention may be selected from the group consisting of: C₁₋₆ alkanols such as methanol, ethanol, n-propanol, isopropanol, n-butanol, sec-butanol, tert-butanol, n-pentanol; cyclopentanol; cyclohexanol; diols, particularly diols having from 2 to 12 carbon atoms such as ethylene glycol, propylene glycol, butylene glycol, 1,5-pentanediol, pentylene glycol, hexylene glycol but also including thiodiglycol and oligo- and poly-alkyleneglycols, such as diethylene glycol, triethylene glycol, dipropylene glycol, polyethylene glycol and polypropylene glycol; triols such as 1,2,6-hexanetriol; ketones and ketone-alcohols, such as acetone, methyl ethyl ketone, 2-pentanone, 3-pentanone, methyl isobutyl ketone, cyclohexanone and diacetone alcohol; tetrahydrofuran; dioxane; mono-C₁₋₄-alkyl ethers of diols having from 2 to 12 carbon atoms, such as ethylene glycol mono-(C₁-C₄)-alkyl ethers, propylene glycol mono-(C₁-C₄)alkyl ethers and in particular ethylene glycol monomethyl ether, ethylene glycol monobutyl ether, propylene glycol monomethyl ether, propylene glycol monopropyl ether, propylene glycol monobutyl ether; diethylene glycol mono-(C₁-C₄)alkyl ethers, such as diethylene glycol monomethyl ether and diethylene monobutyl ether; dipropylene glycol mono-(C₁-C₄)alkyl ethers, such as dipropylene glycol Npropyl ether, dipropylene glycol monopropyl ether and dipropylene glycol monobutyl; propylene glycol phenyl ether; linear amides, such as N,N-dimethylformamide and N.N-dimethylacetamide; cyclic amides such as 2-pyrrolidone, Nmethyl-2-pyrrolidone, N-ethyl-2-pyrrolidone, caprolactam and 1,3-dimethylimidazolidone; sugar esters such as dimethyl isosorbide; cyclic esters such as caprolactone; and, sulfoxides, such as dimethyl sulfoxide and sulfolane.

Said at least one water-miscible solvent is preferably selected from the acetone, diacetone alcohol, isopropyl alcohol and mixtures thereof.

The presence of water-immiscible organic solvents in this formulation is not strictly precluded but it does not represent a preferred embodiment. Rather it is preferred that the formulation is essentially free of water-immiscible organic solvents and as such presents as a single, continuous aqueous phase.

As regards component d) mentioned above, binders or binding agents do represent customary ingredients of aqueous tattoo ink compositions which may be present in an amount up to 5 wt.-%, based on the weight of the composition. The binders are non-volatile ingredients which bind particulate colorants to each other and, in doing so, facilitate the intra-dermal or intra-epidermal introduction of the ink using needles and like injection means. Exemplary binders, which may be used alone or in combination, include but are not limited to: polyvinylpyrollidone (PVP), in particular polyvinylpyrollidone having a weight average molecular weight of from 1 to 3000 kDa, polyethylene glycols (PEG), in particular polyethylene glycols having a weight average molecular weight of from 0.2 to 6 kDa, polypropylene glycol (PPG) in particular polypropylene glycols having a weight average molecular weight of from 0.2 to 6 kDa, PEG/PPG-block copolymers, in particular PEG/PPG block copolymers having a weight average molecular weight of from 5 to 15 kDa, PEG/PPG-random copolymers, and Shellac resin.

The aqueous tattoo ink compositions will typically comprise up to 5 wt.-%, for example up to 3 wt.-%, based on the weight of the composition, of surfactant. A wide variety of surfactants may be used herein - both for emulsification of the dispersed phase and to provide acceptable spreading of the applied composition - but desirable surfactants will be selected from the group consisting of: anionic surfactants, nonionic surfactants, amphoteric surfactants, non-lathering surfactants, emulsifiers, and mixtures thereof. The disclosure regarding surfactants provided for the aqueous hair dyeing composition above is considered applicable and is incorporated herein by reference.

Examples of pH adjusters: mineral acids such as phosphoric acid, hydroxycarboxylic acids such as lactic acid, glycolic acid, citric acid, 3-hydroxybutyric acid, 4-hydroxybutyric acid, 2-hydroxybutanedioic acid, 2,3dihydroxybutanedioic acid, 3-hydroxyvaleric acid, 5-hydroxyvaleric acid, 6-hydroxycaproic acid and the alkali metal or alkaline earth metal salts thereof, ammonia, alkali metal hydroxides, alkali metal carbonates, alkali metal bicarbonates, alkaline earth metal hydroxides, alkaline earth metal carbonates, and alkaline earth metal bicarbonates.

The formulation of this embodiment may comprise from 0 to 5 wt.-%, for example from 0 to 3 wt.-%, based on the weight of the composition, of one or more thickening agents. Said thickening agents may be selected from: i) carboxylic acid polymers, such as those members of the CARBOPOL^{®}900 series available from B.F. Goodrich; ii) acrylates/C10-C30 alkyl acrylate crosspolymers, such as CARBOPOL^{®}1342, CARBOPOL^{®}1382, PEMULEN^{®} TR-1 and PEMULEN^{®} TR-2 available from B.F. Goodrich; iii) crosslinked polyacrylate polymers, which may be cationic or nonionic polymer; iv) polyacrylamide polymers, in particular nonionic polyacrylamide polymers; iv) multi-block copolymers of acrylamides and substituted acrylamides with acrylic acids and substituted acrylic acids; v) polysaccharides including scleroglucans, cellulose and cellulose derivatives, such as carboxymethylcellulose and alkyl hydroxyalkyl cellulose ethers; vi) modified starches; vii) gums such as xantham gum, acacia gum, alginin, sodium alginate, locust bean gum and guar gum; viii) proteins such as collagen, albumen and gelatin; and, mixtures thereof.

As is known in the art, humectants, or moisturizing agents, are cosmetic ingredients with water binding properties that are capable of retaining large amounts of water relative to their weight. Humectants are usually more soluble in water than in oil. Instructive references on suitable humectants for use in the present formulation include WO98/22085, WO98/18444 and WO97/01326. And exemplary humectants for use herein include: amino acids, collagen amino acids or peptides, keratin amino acids, silk amino acids, urea, glycosaminoglycans, N-acetyl glucosamine, glycerine, polyethylene glycol ethers of glycerine, and alkali metal salts of hyaluronic acid, acetylhyaluronic acid, aspartic acid, glucuronic acid and glutamic acid.

The formulation of this embodiment may comprise from 0 to 5 wt.-%, preferably from 0 to 2 wt.-%, based on the weight of the composition, of at least one antimicrobial agent. It is preferred that the or each antimicrobial agent included in the composition is a crystalline particulate that is insoluble in water. Exemplary antimicrobial agents include but are not limited to: sulphur, piroctone olamine, selenium sulfides, as described in US Patent No. 2,694,668, US Patent No. 3,152,046 and US Patent No. 4,089,945, and pyridinethione salts, as described in US Patent No. 3,753, 196, US Patent No. 4,345,080, US Patent No. 4,323,683, and US Patent No. 4,470,982.

The formulation of this embodiment may comprise from 0 to 2 wt.-%, based on the weight of the composition, at least one anti-inflammatory agent. Suitable anti-inflammatory agents, which may be used alone or in combination, include: vitamin F, vitamin E, unsaturated fatty acids, rutin, bioflavonoids, caffeic acid phenethyl ester, sea buckthorn oil, olive oil, jojoba oil, chamomile essential oil, chamomile extract, witch hazel (Hamamelis virginiana) extract, beetroot extract, horseradish extract, dandelion extract, Gamguk extract (Chrysanthemum indicum), Sukbagui extract, betamethasone, dexamethasone, and mixtures thereof. The aforementioned extracts may be obtained by known methods including hot water or alcoholic extraction processes. A preference for witch hazel extract might be mentioned.

Illustrative antioxidants include vitamin E, vitamin F, vitamin C, rutin, resveratrol, carnosic acid, chitosan, flavonoids, gallates, anthocyanins and carotenoids. Illustrative antiseptics include captan, chlorhexidine, hexachlorophene, triclosan, triacetin and mixtures thereof. In certain embodiments, the formulation of this embodiment may comprise up to 1 wt.-%, based on the weight of the composition, of at least one local anesthetic. As is known in the art, local anesthetics reduce the excitability of sensitive nerve fibers, block the influx of sodium ions through interaction with a binding site on the inside of the membrane of the sodium ion channel and thus prevent the development of action potentials necessary for conduction of excitation. Herein preferred local anesthetics are selected from the group consisting of lidocaine, mepivacaine, prilocaine, articaine, bupivacaine, dibucaine, ropivacaine, etidocaine, dyclonin, procaine, benzocaine, 2-chloroprocaine, oxybuprocaine, tetracaine, fomocaine, etidocaine, pramocaine, Levobupivacaine, oxyprocaine, hexylcaine, dibucaine, piperocaine, butamben, butamben picrate, dimethisoquine hydrochloride, diperodone, dyclonine, ketamine, p-butylaminobenzoic acid, pramoxine and their pharmaceutically acceptable salts and mixtures thereof. A preference for lidocaine and benzocaine may be mentioned: this preference is intended to encompass the pharmaceutically acceptable salts of these compounds.

The tattoo ink compositions of the present disclosure may be applied by any conventional technique. As will be recognized by the skilled artisan, the application of the tattoo may be preceded by preparation of the skin by cleaning to remove surface contaminants and / or the application of an anesthetic to provide local and temporary pain relief. The pre-application of a local anesthetic may compliment any anesthetic included within the ink composition itself, as described above.

An intra-epidermal tattoo may be regarded as a "semi-permanent colorant" in that the colorant is disposed within the epidermis but does not diffuse out of the skin and cannot be removed therefrom skin without either the physical disruption or the natural desquamation of the skin. The intra-epidermal tattoo may not, in particular, be washed off under the action or water, soap, alcoholic solvents or combinations thereof.

Epidermal administration of the compositions of the present disclosure may be effected by, for example: needle; microneedle; jet injection; thermal microporation; electroporation; sonoporation; or, combinations thereof. Moreover, the locus of epidermal administration may be to one or more of the stratum corneum, stratum lucidum, stratum granulosum, stratum spinosum and stratum basale.

Without intention to limit the present disclosure, it is considered that techniques for intra-dermal application should be characterized by the introduction of the ink into the dermis under capillary action using a needle which possesses at least three fine points, which points move backwards and forward axially at a frequency of from 50 to 5000 min⁻¹. Under such a mode of introduction, macrophages are released within the dermis as a defensive reaction: given, however, that the ink particulates are much larger than the macrophages, the consequent effect of the ink particulates being surrounded by the phagocytic cells is to fix the ink particulates within the dermis. Under such a mode of introduction, it is also established that a fraction of the ink particulates will be taken up by fibroblasts and a further fraction will be retained within the extracellular matrix of collagen fibers in the dermis. Any ink deposited within the epidermis will however be lost given that epidermal cells are shed both naturally and in a healing response to the injury caused by the needle.

Exemplary tattoo needles for intra-dermal applications include but are not limited to those described in: EP 2454966 A1; US 2007/0038181 A (Melamud); US 2004/0186501 A (Kuei); United States Patent No. 8,764,784 (Crockett); US Design Patent No. 866950 S1 (Schubert); and, US Design Patent No. 888240 S1 (Importla).

Whilst tattoos are intended to be permanent, it is recognized that persons do often seek to remove them from their bodies. The use of melanoidins as pigments is considered to present the benefit that these compounds can be degraded through the targeted action of enzymes and chemicals, such as hydrogen peroxide and ozone, introduced into the dermis or epidermis. The degradation of the melanoidins renders the pigment "removable".
Cosmetic embodiment 10
   is a solid cosmetic composition, said composition comprising:
   a) at least one colorant, wherein said at least one colorant comprises a nanoscale melanoidin product, said nanoscale melanoidin product having a weight average molecular weight of from 200 daltons to 300 kDa, preferably from 1 to 300 kDa and more preferably from 1 to 100 kDa;
   b) at least one structurant; and
   c) at least one thickener.
Cosmetic embodiment 11
   is a solid cosmetic composition comprising, based on the weight of the composition:
   a) from 2 to 30 wt.-% of at least one colorant, wherein said at least one colorant comprises a nanoscale melanoidin product, said nanoscale melanoidin product having a weight average molecular weight of from 200 daltons to 300 kDa, preferably from 1 to 300 kDa and more preferably from 1 to 100 kDa;
   b) from 4 to 50 wt.-% of at least one structurant;
   c) from 4 to 50 wt.-% of at least one thickener; and
   d) from 0 to 90 wt.% of at least one auxiliary selected from the group consisting of: carriers, stabilizers, surfactants, and preservatives.

Component d) in cosmetic embodiment 11 represents categorizations based on the benefit provided by the agent or the agent's postulated mode of action. This categorization is made for convenience: a given chemical ingredient might provide more than one benefit or operate via more than one mode of action.

The solid cosmetic composition may comprise from 4 to 50 wt.-%, for example from 5 to 40 wt.-% or from 5 to 30 wt.-%, based on the weight of the composition, of one or more structurants. Without intention to limit the present invention, said structurants having utility in the present invention should comprise or consist of waxes having a softening point of from 50 to 150°C and may include one or more of: i) polyethylene having a number average molecular weight from 500 to 7500; ii) petroleum waxes, such as paraffin wax, ozokerite wax, ceresin wax, aderwax, earth wax and microcrystalline wax; iii) synthetic waxes made by polymerizing carbon monoxide and hydrogen, such as Fischer-Tropsch wax; iv) polyolefin waxes; v) hydrogenated animal, fish or vegetable oils; and, vi) plant and animal derived waxes, such as beeswax, carnauba wax, candelilla wax, spermeceti wax and baysberry wax.

The solid cosmetic composition may comprise from 4 to 50 wt.-%, for example from 5 to 40 wt.-% or from 5 to 30 wt.-%, based on the weight of the composition, of one or more thickening agents. Said thickening agents may be selected from: i) carboxylic acid polymers, such as those members of the CARBOPOL^{®}900 series available from B.F. Goodrich; ii) acrylates/C10-C30 alkyl acrylate crosspolymers, such as CARBOPOL^{®}1342, CARBOPOL^{®}1382, PEMULEN^{®} TR-1 and PEMULEN^{®} TR-2 available from B.F. Goodrich; iii) crosslinked polyacrylate polymers, which may be cationic or nonionic polymer; iv) polyacrylamide polymers, in particular nonionic polyacrylamide polymers; iv) multi-block copolymers of acrylamides and substituted acrylamides with acrylic acids and substituted acrylic acids; v) polysaccharides including scleroglucans, cellulose and cellulose derivatives, such as carboxymethylcellulose and alkyl hydroxyalkyl cellulose ethers; vi) modified starches; vii) gums such as xantham gum, acacia gum, alginin, sodium alginate, locust bean gum and guar gum; viii) proteins such as collagen, albumen and gelatin; and, mixtures thereof.

The solid cosmetic composition may comprise up to 50 wt.-% of carriers which have the functionality of leaving behind, upon application, a film comprising the nanoscale melanoidin product as the colorant. Exemplary carriers include: water; water-miscible solvents; water-immiscible solvents; volatile silicones, as described in Todd et al., "Volatile Silicone Fluids for Cosmetics", Cosmetics and Toiletries, 91:27-32 (1976); non-volatile organic fluids; and, non-volatile silicone fluids. The term "volatile silicone" refers to those silicone materials that have measurable vapor pressure under ambient conditions: cyclomethicone is an important example.

For completeness, non-limiting examples of nonvolatile organic fluids include mineral oil, PPG-14 butyl ether, isopropyl myristate, petrolatum, butyl stearate, cetyl octanoate, butyl myristate, myristyl myristate, C12-15 alkylbenzoate (e.g., Finsolv.TM.), dipropylene glycol dibenzoate, PPG-15 stearyl ether benzoate and blends thereof (e.g. Finsolv TPP), neopentyl glycol diheptanoate (e.g. Lexfeel 7 supplied by Inolex), octyldodecanol, isostearyl isostearate, octododecyl benzoate, isostearyl lactate, isostearyl palmitate, isononyl/ isononoate, isoeicosane, octyldodecyl neopentanate, hydrogenated polyisobutane, and isobutyl stearate. US Patent No. 6,013,248 (Luebbe et al.) and US Patent No. 5,968,489 (Swaile et al) are considered to provide instructive references in this context.

The solid cosmetic compositions will typically comprise up to 5 wt.-%, for example up to 3 wt.-%, based on the weight of the composition, of surfactant. A wide variety of surfactants may be used herein - both for emulsification of the dispersed phase and to provide acceptable spreading of the applied composition - but desirable surfactants will be selected from the group consisting of: anionic surfactants; nonionic surfactants; amphoteric surfactants; non-lathering surfactants; emulsifiers; and, mixtures thereof. The disclosure regarding surfactants provided for the aqueous hair dying composition above is considered applicable and is incorporated herein by reference.

A preservative may also be present in the solid cosmetic composition. Examples thereof include: phenoxy ethanol; C1-C6 alkyl parabens; imidazolinyl urea; dimethydimethoyl hydantoin; N-(3-chloroallyl) hexaminium chloride; cetrimonium bromide; trisodium ethylene diamine tetraacetic acid; and, butylated hydroxyanisole (BHA). When present, the preservative will typically be added in an amount up to 0.5 wt.-%, for instance from 0.01 to 0.1 wt.-%, based on the weight of the composition. However, given the preservative functionality of melanoidins, it is also envisaged that said solid cosmetic compositions may be substantially free of preservatives.

The solid cosmetic compositions of the present invention can be formulated as any known or otherwise effective product form for providing topical application of the melanoidin active to the desired area of the skin. Non-limiting examples of such product forms include cakes, sticks, pencils and roll-ons, subject to the proviso that the selected form contains all the essential elements as defined herein. The solid compositions are generally stored in and dispensed from a suitable package or applicator device which should be closeable to prevent loss of any constituent volatile compounds prior to and between applications.

The solid cosmetic compositions according to cosmetic embodiments 10 and 11 are especially well suited as facial make-up.

The use of melanoidin nanoparticles or nanofibers in cosmetic compositions has several advantages over known cosmetic compositions:
By the direct inclusion of nanoscale melanoidins in cosmetic compositions said melanoidins can replace - in whole or in part - organic pigments, mineral pigments and synthetic dyes commonly employed. This is beneficial in view of potential health problems and detrimental effects associated with known pigments and dyes, for instance for carbon black.

Nanoscale melanoidins obtained from coffee grounds, especially from spent coffee grounds, are an environmentally friendly, renewable and sustainable source for cosmetic composition ingredients.

Nanoscale melanoidins in cosmetic compositions have the ability to intrude into the skin and better adhere to the skin. This effect can advantageously be used for instance in sunscreen, skin colorant or hair colorant applications.

### Detailed description of exemplary embodiments of the invention

In the following, further preferred embodiments of the invention are illustrated by means of examples. The invention is not limited to these examples, however. The features as described in the above description, the following examples and the claims can be relevant individually or in any combination to realise the various embodiments of the invention.

The following methods of analysis were used if not noted otherwise: Concentrations in solutions were determined by absorbance by UV-VIS at 405 nm and various wavelengths. Molecular weight distributions were determined by gel permeation chromatography and/or gas chromatography and mass spectrometry. Electrical surface, surface pH and stability related to surface charge were determined by zeta potential measurements.

### Example 1

A melanoidin product has been prepared by a process comprising an extraction step, a first separation step, a precipitation step and a second separation step according to the invention.

### Extraction step

The extraction step was performed in a stainless steel reactor with an internal volume of 1.2 liter. The reactor was equipped with a mechanical stirrer inside the reactor volume, an external electrical heating and a serpentine cooling coil inside the reactor. The reactor was preheated to 80°C by the electrical heating. An amount of 100 g of spent coffee grounds powder was put into the reactor.

The extraction agent was prepared by dissolving 30 g of sodium hydroxide (NaOH) in 1 liter of distilled water. The pH value of the extraction agent was 13.9. The extraction agent was preheated to a temperature of 80°C and fed into the reactor immediately after the coffee grounds powder. The reactor was closed and its content was stirred at a speed of from 50 to 100 rpm. The reactor was heated for 15 min until a temperature of 120°C was reached. The pressure inside the reactor rose to 3 bars (abs). That temperature was maintained for another 30 min. The heating was then switched off and a liquid cooling fluid was flown through the cooling coils. The liquid contents of the reactor was rapidly cooled down to 40°C in 10 min. The reactor was opened and the resulting melanoidin-containing solute in the fluid phase of the extraction agent was obtained.

### First separation step

The contents of the reactor was fed to a filter press with a cut-off at 200 µm. The fluid phase comprising the melanoidin-containing solute was separated from the coffee grounds which were discharged.

### Precipitation step

The fluid phase separated in the first separation step was fed to a 2 liter glass beaker. Hydrochloric acid (HCI) at 37% (w/v) concentration was added drop by drop to the fluid phase until a pH value of 2 was measured. The pH value was continuously measured by a pH electrode. During the acidification, an increase of turbidity of the acidic mixture was observed, indicating the formation of precipitates.

### Second separation step

The acidic mixture rested for about 12 hours. The acidic mixture was then homogenized by stirring and fed to a centrifuge (model 5810R by company Eppendorf). After centrifugation at 10,000 rpm for 10 min the solid particles were retrieved in 50 ml flasks. The liquid phase was discharged. The solid particles in the flasks were re-suspended with 50 ml of acidified water and submitted to centrifugation for a second time. The solid particles obtained from the second centrifugation were re-suspended in water and freeze-dried. The resulting powder had a dark brown colour and amounted to 21 wt.-% of the initial coffee ground powder.

### Example 2

1 g of a powder obtained by the method according to Example 1 was dissolved in 50 ml (milliliter) of demineralized water. The slightly acidic solution was neutralized with sodium hydroxide to a pH value of 7. The neutralized mixture was fed to an ultrafiltration membrane where a fraction of particles with a molecular weight of more than 300 kDa was separated from a fraction with lower molecular weight. The high molecular weight fraction was mixed with an aqueous solution of ethanol. The aqueous solution contained 75% by volume of ethanol and 25% by volume of water. 80% by weight of the aqueous ethanol solution was mixed with 20% by weight of the melanoidin containing mixture.

The resulting mixture was fed to an electrospinning apparatus. The target was an aluminium foil. The distance between the needle and the target was 15 cm. The voltages applied were varied between 10 kV and 20 kV. By this process melanoidin containing nanofibers with a diameter of from 300 to 400 nm (nanometres) were obtained.

### Example 3

1 g of a powder obtained by the method according to Example 1 was dissolved in 50 ml of demineralized water. The slightly acidic solution was neutralized with sodium hydroxide to a pH value of 7. The neutralized mixture was fed to an ultrafiltration membrane where a fraction of particles with a molecular weight of more than 100 kDa was separated from a fraction with lower molecular weight. The high molecular weight fraction was mixed with an aqueous solution of ethanol. The aqueous solution contained 50% by volume of ethanol and 50% by volume of water. 84.15% by weight of the aqueous ethanol solution was mixed with 15% by weight of the melanoidin containing mixture and with 0.85% by weight of pure polyvinyl alcohol with a molecular weight (Mw) of 85000 to 124000, 87-89% hydrolized (Sigma Aldrich).

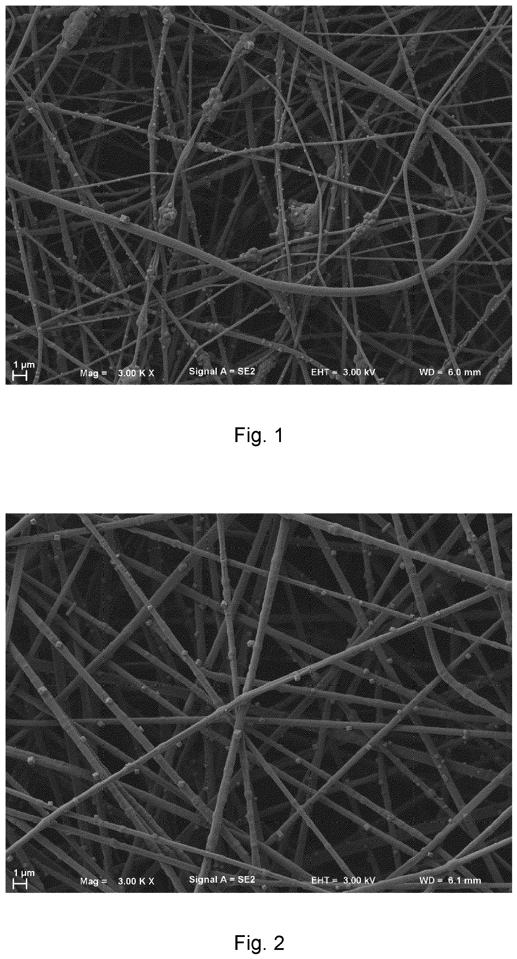

The resulting mixture was fed to an electrospinning apparatus at a flow rate of 0.3 ml/h. The target was an aluminium foil. The distance between the needle and the target was 12 cm. The voltages applied was 13.5 kV. By this process a multilayer fiber mat was obtained. Fig. 1 shows a picture of the obtained fibers taken from a scanning electron microscope (SEM). The scale is given in the lower left corner of Fig. 1 as 1 µm. The diameter of the fibers was in the range of from 200 nm to 500 nm.

### Example 4

1 g of a powder obtained by the method according to Example 1 was dissolved in 50 ml of demineralized water. The slightly acidic solution was neutralized with sodium hydroxide to a pH value of 7. The neutralized mixture was fed to an ultrafiltration membrane where a fraction of particles with a molecular weight of more than 100 kDa was separated from a fraction with lower molecular weight. The high molecular weight fraction was mixed with an aqueous solution of ethanol. The aqueous solution contained 50% by volume of ethanol and 50% by volume of water. 82.45% by weight of the aqueous ethanol solution was mixed with 15% by weight of the melanoidin containing mixture and with 2.55% by weight of pure polyvinyl alcohol with a molecular weight (Mw) of 85000 to 124000, 87-89% hydrolized (Sigma Aldrich).

The resulting mixture was fed to an electrospinning apparatus at a flow rate of 0.3 ml/h. The target was an aluminium foil. The distance between the needle and the target was 12 cm. The voltages applied was 11.7 kV. By this process a multilayer fiber mat was obtained. Fig. 2 shows a picture of the obtained fibers taken from a scanning electron microscope (SEM). The scale is given in the lower left corner of Fig. 2 as 1 µm. The diameter of the fibers was in the range of from 250 nm to 450 nm.

### Example 5

1 g of a powder obtained by the method according to Example 1 was dissolved in 50 ml of demineralized water. The slightly acidic solution was neutralized with sodium hydroxide to a pH value of 7. The neutralized mixture was fed to an ultrafiltration membrane where a fraction of particles with a molecular weight of more than 100 kDa was separated from a fraction with lower molecular weight. The high molecular weight fraction was mixed with an aqueous solution of ethanol. The aqueous solution contained 50% by volume of ethanol and 50% by volume of water. 80% by weight of the aqueous ethanol solution was mixed with 20% by weight of the melanoidin containing mixture.

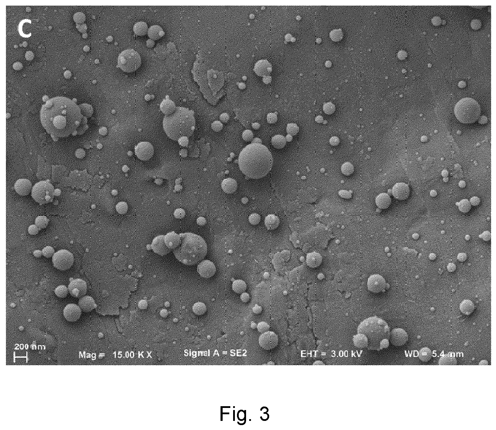

The resulting mixture was fed to an electrospinning apparatus. The target was an aluminium foil. The distance between the needle and the target was 16 cm. The voltages applied was 24.7 kV. By this process spherical melanoidin-containing nanoparticles were obtained. Fig. 3 shows a picture of the obtained particles taken from a scanning electron microscope (SEM). The scale is given in the lower left corner of Fig. 3 as 200 nm. The particles were smaller than 400 nm in diameter and had a smooth surface.

### Example 6

A melanoidin product has been prepared by a process comprising an extraction step, a first separation step, a precipitation step and a second separation step according to the invention.

The extraction step and the first separation step were identical to that of Example 1.

### Precipitation step

The fluid phase separated in the first separation step was fed to a vessel with a mechanical stirrer inside the vessel volume. The fluid phase was at ambient temperature (around 25°C). The stirrer was operated at 400 to 500 rpm. The fluid phase was contacted with ethyl acetate as an organic phase separation agent. Ethyl acetate was added to the fluid phase in an amount corresponding to 25% of the volume of the fluid phase. The stirring was continued until an emulsion of the two immiscible liquids had formed. Hydrochloric acid (HCI) at 37% (w/v) concentration was slowly added to the emulsion until a pH value of 7 was measured. The pH value was continuously measured by a pH electrode. During the acidification, an increase of turbidity of the mixture was observed, indicating the formation of precipitates.

### Second separation step

The mixture was then fed to a centrifuge (model 5810R by company Eppendorf). After centrifugation at 2,000 rpm for 5 min the mixture was filled in a flask. After settlement three phases were separated: an aqueous phase at the bottom of the flask, an ethyl acetate phase on the top and a intermediate phase containing the melanoidin containing precipitates. Solid particles were retrieved from the intermediate phase by filtration and subsequently dried in an oven. The resulting powder had a dark brown colour and amounted to 21 wt.-% of the initial coffee ground powder. The powder was analysed for its particle size distribution. The particle size was between 40 and 600 nm (nanometres) with the majority of the particles being between 200 and 400 nm.

### Example 7

A melanoidin product has been prepared by the method according to Example 1. The product obtained was further processed by ultrafiltration, resulting in fractions of different molecular weights: 300 kDa, 100 kDa, 30 kDa, 10 kDa, 3 kDa, 1 kDa and less than 1 kDa. The membranes (Ultracel^{®} by Merck) used were of regenerated cellulose and 76 mm in diameter. The different fractions were recovered from the solution by freeze drying. The yields were calculated as percentages of the total melanoidin content.

The total phenolic content (TPC) of the respective melanoidin fraction was determined by the Folin-Ciocalteu method. Each extract was first diluted in deionized water or in NaOH 0.01 M solution when the extract was not soluble in pure water. Aliquots of 50 µL of sample and 500 µL of Folin-Ciocalteu reagent were mixed for 15 s and incubated at room temperature for 30 min. Then, 450 µL of 75 g/L sodium carbonate was added to the mixture. After 1 h of storage in the dark at room temperature, the absorbance at 760 nm of the mixture was measured using a microplate reader. Standard solutions of trihydroxybenzoic acid containing known concentrations of gallic acid were used to determine a calibration and the results were expressed in terms of mg gallic acid equivalent (mg GAeq) per mass. Each analysis was performed in triplicate and averaged.

The total protein content was determined using the Bradford protein assay. 10 µl of the filtered extracts duly diluted in the same solvent used for extraction were mixed with 300 µl of Coomassie Blue reagent in a 96-well microplate. The microplate was then stirred for 30 s and, after 10 min at room temperature, the absorbance was measured at 595 nm in a spectrophotometric microplate reader (Sunrise Tecan, Grödig, Austria), using a blank prepared with distilled water. A calibration curve was prepared using aqueous solutions of BSA (bovine serum albumin). The protein content was expressed as milligram per mass of material (mg BSA/g).

Table 1 below shows the yields, total phenolic contents (TPC) and total protein contents of the different fractions.

**Table 1**

| Membrane pore size (kDa) | Yield (% of melanoidins fraction) | TPC (mg GAeq / g) | Total protein (mg BSAeq / g) |
|---|---|---|---|
| 300 | 10.1 | 39.7 | 206.7 |
| 100 | 7.6 | 53.4 | 274.2 |
| 30 | 6.6 | 71.9 | 328.3 |
| 10 | 20.7 | 88.1 | 344.7 |
| 3 | 33.1 | 107.4 | 221.6 |
| 1 | 5.1 | 71.5 | 54.6 |
| <1 | 16.8 | 55.3 | 397.3 |

### Example 8

The conductivity of melanoidin nanoparticles as an electrolyte solution was measured at room temperature. Demineralized water was used as the basis for the solution. The conductivity of the base solution without melanoidin nanoparticles was 15 µS/cm (10⁻⁶ Siemens / centimetre). Melanoidin nanoparticles with a weight fraction in the range from 10 kDa to 300 kDa were added to the base solution. The solution was thoroughly stirred before taking the conductivity measurements. Table 2 below shows the measured conductivities for the different concentrations of melanoidin nanoparticles in solution.

**Table 2**

| Melanoidin concentration (mg/ml) | Conductivity (µS/cm) |
|---|---|
| 0.5 | 1112 |
| 1 | 2160 |
| 5 | 5440 |
| 10 | 6950 |

## Claims

1. A process for preparing a melanoidin product, the process comprising:
a) an extraction step in which coffee grounds are treated with an extraction agent with a pH value greater than 7 to extract a melanoidin-containing solute in a fluid phase of the extraction agent;
b) a first separation step in which the fluid phase is separated from the coffee grounds;
c) a precipitation step in which the fluid phase is contacted (i) with an acid to obtain an acidic mixture with a pH value lower than 4 or (ii) with an organic phase separation agent and with an acid to obtain a mixture with a pH value in the range of from 4 to 8, thereby forming precipitates containing melanoidin; and
d) a second separation step in which the formed precipitates are separated from the acidic mixture.

2. The process according to claim 1, wherein the extraction agent is aqueous and comprises sodium hydroxide or potassium hydroxide.

3. The process according to claim 1 or 2, wherein the organic phase separation agent is selected from the group of ethanol, ethyl acetate, tetrahydrofuran and 2-methyltetrahydrofuran.

4. The process according to any of claims 1 to 3, wherein the second separation step comprises a fractionation step in which a low-molecular weight fraction of the formed precipitates with an average molecular weight of less than 10 kDa is obtained, and wherein the second separation step is followed by a nanoparticle production step in which nanoparticles are produced from the low-molecular weight fraction.

5. The process according to claim 4, wherein the nanoparticle production step comprises at least one method selected from the group of electrospinning, laser ablation, freeze drying, spray drying, convective drying, radiation drying, and vacuum drying.

6. The process according to any of claims 1 to 3, wherein the second separation step comprises a fractionation step in which a high-molecular weight fraction of the formed precipitates with an average molecular weight of at least 10 kDa is obtained, and wherein the second separation step is followed by a nanofiber production step in which nanofibers are produced from the high-molecular weight fraction.

7. The process according to claim 6, wherein the nanofiber production step comprises electrospinning.

8. A melanoidin product obtained by a process according to any of claims 1 to 7.

9. The melanoidin product according to claim 8, wherein the melanoidin product is a melanoidin nanoparticle having an average diameter of 1 nm to 1000 nm or a melanoidin nanofiber having an average diameter of 1 nm to 1000 nm.

10. A melanoidin nanoparticle having an average diameter of 1 nm to 1000 nm and comprising more than 70 wt.-% of melanoidins, wherein the melanoidins comprise from 1 wt.-% to 15 wt.-% of phenolic compounds and/or from 1 wt.-% to 40 wt.-% of proteins.

11. A melanoidin nanofiber having an average diameter of 1 nm to 1000 nm and comprising more than 50 wt.-% of melanoidins, wherein the melanoidins comprise from 1 wt.-% to 15 wt.-% of phenolic compounds and/or from 1 wt.-% to 40 wt.-% of proteins.

12. An electronic component comprising a melanoidin product according to any of claims 8 to 11.

13. A conductive liquid comprising a melanoidin product according to any of claims 8 to 11.

14. Use of a melanoidin product according to any of claims 8 to 11 for a photothermal application, in particular photothermal therapy, photodynamic therapy, photothermal catalysis, photothermal antibacterial treatment, solar collectors, solar heating, solar evaporation.

15. A cosmetic composition comprising a melanoidin product according to any of claims 8 to 11.
